## Europäisches Patentamt
## European Patent Office
## Office européen des brevets

(11) Veröffentlichungsnummer: **0 133 553**
**A2**

# (12) EUROPÄISCHE PATENTANMELDUNG

(21) Anmeldenummer: **84109177.0**

(22) Anmeldetag: **02.08.84**

(51) Int. Cl.⁴: **G 01 N 33/53**

(30) Priorität: **10.08.83 DE 3328832**

(43) Veröffentlichungstag der Anmeldung:
**27.02.85 Patentblatt 85/9**

(84) Benannte Vertragsstaaten:
**AT BE CH DE FR GB IT LI LU NL SE**

(71) Anmelder: **BOEHRINGER INGELHEIM DIAGNOSTIKA GMBH**
**Gutenbergstrasse 3 Postfach 1609**
**D-8046 Garching bei München(DE)**

(72) Erfinder: **Kolb, Helmut, Prof. Dr.**
**Kranzhornstrasse 13**
**D-8000 München 82(DE)**

(72) Erfinder: **Schleifer, Karl Heinz, Prof. Dr.**
**Schwalbenstrasse 3a**
**D-8044 Lohhof(DE)**

(72) Erfinder: **Seidl, Hans Peter, Dr.**
**Marsopstrasse 20**
**D-8000 München 60(DE)**

(72) Erfinder: **Tympner, Klaus Dieter, Prof. Dr.**
**Waterloostrasse 70**
**D-8000 München 71(DE)**

(72) Erfinder: **Weiss, Ludwig, Prof. Dr.**
**Hauberrisser Strasse 7**
**D-8000 München 90(DE)**

(54) **Reagenz und Verfahren zur quantitativen Bestimmung von Antikörpern.**

(57) Die Erfindung betrifft Antikörper aus der Klasse der Immunglobuline A, M und E gegen das Peptiduntereinheit-Pentapeptid L-Ala-D-Glu(L-Lys-D-Ala-D-Ala) des Peptidoglycans in biologischem Untersuchungsmaterial sowie gegen diese Peptidoglycane enthaltende bakterielle Zellwände, gekennzeichnet durch

a) spezifische Bindung an Pentapeptide der allgemeinen Formel

$$R - A_1 - D\text{-Ala} \qquad (I)$$

worin
R ein Aminosäureanteil mit 1 − 3, vorzugsweise 3 Aminosäuren aus der Gruppe Gly, L-Ala, D-Glu, L-lys und $A_1$ eine der Aminosäuren D-Ala, D-Ser, D-Val oder D-Leu bedeuten, oder

b) spezifische Bindung an Pentapeptide der allgemeinen Formel I enthaltende isolierte bakterielle Zellwände oder

c) spezifische Bindung an Pentapeptide der allgemeinen Formel I enthaltende grampositive Bakterien,

Verfahren zu ihrer Herstellung, Verfahren zu ihrer quantitativen Bestimmung und Reagenz.

Wie bereits im DBP ... (deutsche Patentanmeldung
P 32 31 204.0) angeführt, stellen Antikörper gegen das
Peptiduntereinheit-Pentapeptid L-Ala-D-Glu(L-Lys-D-Ala-
D-Ala) von Peptidoglycan Indikatoren für akut oder
chronisch ablaufende bakterielle Infektionen dar.

Wie neuerdings gefunden wurde, treten neben den im
späteren Stadium einer Infektion nachzuweisenden
spezifischen Antikörpern der Immunglobulinklasse IgG
auch spezifische Antikörper der Immunglobulinklassen
IgA, IgM und IgE auf.

So finden sich Antikörper der Klasse IgM in der Anfangsphase einer Infektion, zum Teil bereits vor dem Auftreten klinischer Symptome. Antikörper der Klasse IgA
sind nachweisbar bei Infektionen, die sich im wesentlichen zuerst an den Schleimhäuten manifestieren.
Antikörper der Klasse IgE finden sich bei Erkrankungen,
die mit allergischen Reaktionen einhergehen.

Die Erfindung betrifft somit Reagenzien zur Bestimmung
von Antikörpern aus der Klasse der Immunglobuline A,
M und E gegen das Peptiduntereinheit-Pentapeptid
L-Ala-D-Glu(L-Lys-D-Ala-D-Ala) des Peptidoglycans sowie
gegen dieses Peptidoglycan enthaltende bakterielle
Zellwände, Verfahren zu ihrer Herstellung und Methoden
zu ihrer quantitativen Bestimmung in biologischem
Untersuchungsmaterial; die Bestimmungsmethoden können
für Routineuntersuchungen verwendet werden.

Die Antikörper sind charakterisiert
a) durch spezielle Bindung an Pentapeptide der allgemeinen Formel

$$R - A_1 - D\text{-}Ala \qquad (I)$$

worin

  R  ein Aminosäureanteil mit 1 - 3, vorzugsweise 3
     Aminosäuren aus der Gruppe Gly, L-Ala, D-Glu,
     L-Lys und

  $A_1$ eine der Aminosäuren D-Ala, D-Ser, D-Val oder
     D-Leu bedeuten; oder

b) durch Bindung an isolierte bakterielle Zellwände,
   die diese Pentapeptide enthalten; oder

c) durch Bindung an grampositive Bakterien, die diese
   Pentapeptide enthalten.

Die Reagenzien werden folgendermaßen erhalten:
An Trägerpolymere (z.B. Albumin) gebundene Pentapeptide
der allgemeinen Formel I werden an eine feste Phase,
z.B. Polystyrol, gekoppelt, mit der Testprobe (z.B.
Patientenserum) inkubiert, gewaschen, mit entsprechenden
Anti-Antikörpern (Anti-IgA, Anti-IgM oder Anti-IgE),
die mit einem Indikatorsystem gekoppelt sind, umgesetzt
und wiederum gewaschen. Sodann kann der Markierungsanteil am festen Träger gemessen werden.
Die Durchführung der Tests zur Bestimmung der verschiedenen Immunglobulinklassen erfordert unterschiedliche
Bedingungen, die in den Beispielen detailliert beschrieben sind.

Die Erfindung betrifft außerdem ein Verfahren zur
quantitativen Bestimmung von Antikörpern gegen das
Peptiduntereinheit-Pentapeptid L-Ala-D-Glu(L-Lys-D-Ala-
D-Ala) des Peptidoglycans in biologischem Untersuchungsmaterial; als solche Verfahren seien genannt der Doppel-
Antikörper-Radioimmun-Assay, der Doppel-Antikörper-
Enzymimmun-Assay, die nephelometrische Methode, der
Festphasen-Sandwich-Radioimmun-Assay, der Festphasen-
Enzymimmun-Assay, der Festphasen-Fluoreszenzimmun-Assay
sowie der Latex-Agglutinationstest.

## Standardisierung der Teste

### 1. Standardgewinnung von spezifischem IgM und IgA

IgM und IgA werden nach konventionellen Methoden
(z.B. FPLC-Chromatographie) aus Seren, die Antikörper mit Spezifität gegen das Peptiduntereinheit-
Pentapeptid der allgemeinen Formel I enthalten,
isoliert und die spezifische Antikörperfraktion
durch Affinitätschromatographie (mit Matrix-gebundenen Peptiden der Formel I) gewonnen. Nach Bindung
der spezifischen Antikörper an die Affinitäts-Matrix
wird nichtgebundenes Serumprotein durch Waschen
mit Pufferlösungen entfernt. Die Elution der an die
feste Matrix gebundenen Antikörper erfolgt durch
spezifische Desorption der gebundenen Immunglobuline
mit Peptiden der allgemeinen Formel I. Die Desorption kann ebenfalls durch im Prinzip veröffentlichte
Techniken wie Anwendung tiefer pH-Werte (wie z.B.
1-molarer Essigsäure) oder hoher Salzkonzentrationen
(wie z.B. 6-molarer Guanidiniumchlorid, 8-molarer
Harnstoff oder 3-molarer NaCl) erfolgen. Nach Dialyse
gegen Pufferlösungen wird der Gehalt an spezifischen
Antikörpern gegen das Peptiduntereinheit-Pentapeptid
des Peptidoglycans über den Immunglobulingehalt der
gereinigten Fraktion quantifiziert.

### 2. Standardisierung der Bestimmung von spezifischem IgE

Diese erfolgt durch Verwendung eines gepoolten Serums
aus Probanden mit hohem spezifischem IgE und anschließendem Vergleich der gemessenen Probe mit einer
Standardkurve (entsprechende Verdünnung).

## 3. Testaufbau

Die quantitative Bestimmung von spezifischen Antikörpern der oben angegebenen Immunglobulinklassen
gegen das Peptiduntereinheit-Pentapeptid des
Peptidoglycans läßt sich mit zwei prinzipiell verschiedenen Methoden durchführen.

a) Quantitative Bestimmung im löslichen System
   (Messung in homogener Phase)

b) Quantitative Bestimmung durch Messung im heterogenen System (Messung in heterogener Phase;
   Festphasen-Immuno-Assay).

3.1. Messung in der homogenen Phase, z.B.

3.1.1. quantitative Bestimmung von Antikörpern gegen das
Peptiduntereinheit-Pentapeptid des Peptidoglycans
in einem Doppel-Antikörper-Radioimmuno-Assay.

Peptide der Formel I werden radioaktiv markiert
und der Testansatz entsprechend nachfolgendem Schema
ausgeführt:

$$\text{*Pept. + Ak} \longrightarrow \text{*Pept.-Ak}$$

Die Trennung von radioaktivem freien Antigen von
radioaktivem Antikörper-gebundenem Antigen erfolgt
nach der Doppel-Antikörpermethode durch spezifische
Präzipitation der Immunglobuline mit Hilfe eines
zweiten Anti-Antikörpers nach folgendem Schema:

$$\text{*Pept.-Ak + Anti-Ak} \longrightarrow \text{*Pept.-Ak}_m\text{-(Anti-Ak)}_n\text{-Komplexe}$$

Die radioaktive Markierung des Antigens kann z.B.
erfolgen

a) durch Kopplung von Tyrosin an das N-terminale
   Ende des Peptids und anschließende Jodierung
   mit $^{125}$J mit konventionellen Methoden

b) durch Jodierung mit (N-Succinimidyl-3-(4-hydroxy,
   5-($^{125}$J)jodophenyl)propionat

c) durch Tritiierung des Antigens mit N-Succinimidyl
   (2,3-$^{3}$H)propionat.


Es ist angebracht, die verwendeten Anti-Antikörper
vor Verwendung durch Affinitätschromatographie an
Matrix gebundenen Peptiden der Formel I zur Entfernung von eventuell enthaltenden Antikörpern gegen
Peptidoglycan zu reinigen bzw. vorzugsweise monoklonale Anti-antikörper zu verwenden.


3.1.2. Quantitative Bestimmung von Antikörpern gegen das
       Peptiduntereinheit-Pentapeptid in einem Doppelantikörper-Enzymimmuno-Assay.

Testaufbau wie unter 3.1.1., jedoch erfolgt die
Markierung der Anti-Antikörper mittels eines Enzyms
wie z.B. alkalische Phosphatase, Peroxidase,
Glucose-Oxidase-Peroxidase, Glucose-6-Phosphat-
dehydrogenase, Malat-Dehydrogenase etc. anstelle von
Radioaktivität.

3.1.3. Quantitative Bestimmung von Antikörpern gegen das
Peptiduntereinheit-Pentapeptid des Peptidoglycans
mittels nephelometrischer Methoden.
Peptide der Formel I werden über die N-terminale
Aminogruppe an eine lösliche Matrix, z.B. Proteine,
Polyvinyl-Pyrrolidon etc. gebunden und der Testansatz entsprechend folgendem Schema ausgeführt:

$$\text{Matrix-(Pept.)}_n + \text{Ak} \longrightarrow \text{Matrix-(Pept.)}_n\text{-Ak-Komplexe}$$

$$\text{Trübung}$$

Die Kopplung der Peptide an das Trägermaterial kann
z.B. erfolgen
1. durch Bindung der jodacetylierten Peptide an die
   Matrix
2. mit der Carbodiimid-Methode
3. mittels bifunktioneller Reagenzien, z.B. Diisocyanate, Glutardialdehyd etc..

3.2. Messung in der heterogenen Phase

Peptide der Formel I werden über ihre N-terminale
Aminogruppe an eine feste, unlösliche Matrix gebunden,
wie z.B. Papier, Kunststoffe, Latex etc.. Die Kopplung erfolgt nach konventionellen Methoden. Die
Bindung der Peptide kann entweder direkt an die
Matrix erfolgen oder über sogenannte "spacer".
Als "spacer" können dienen: aliphatische Kohlenwasserstoffketten, Proteine wie z.B. Albumin,
RNase etc..

3.2.1. Quantitative Bestimmung von Antikörpern gegen das
Peptiduntereinheit-Pentapeptid des Peptidoglycans
in einem Festphasen-"Sandwich"-Radio-Immuno-Assay.

3.2.2. Quantitative Bestimmung von Antikörpern gegen das
Peptiduntereinheit-Pentapeptid des Peptidoglycans
in einem Festphasen-Enzym-Immuno-Assay.

Testaufbau wie unter 3.2.1., jedoch erfolgt die
Markierung der Anti-Antikörper mittels eines
Enzyms wie unter 3.1.2. aufgeführt, anstelle von
Radioaktivität.

3.2.3. Quantitative Bestimmung von Antikörpern gegen das
Peptiduntereinheit-Pentapeptid des Peptidoglycans
in einem Festphasen-Fluoreszenz-Immuno-Assay.

Testaufbau wie unter 3.2.1., jedoch erfolgt die Markierung des Antikörpers mittels eines Fluoreszenz-
Farbstoffes (vgl. auch FIAX® - StiQ™-System).

3.2.4. Quantitative Bestimmung von Antikörpern gegen das
Peptiduntereinheit-Pentapeptid des Peptidoglycans
mittels eines Latex-Agglutinationstestes.

Peptide der Formel I werden entweder direkt oder
über "spacer", wie z.B. Proteine, an Latexpartikel
gekoppelt.

Bei den unter 3.1. sowie unter 3.2. aufgeführten
quantitativen Bestimmungsmethoden erfolgt die
Quantifizierung mittels Eichkurve, die mit Hilfe
der unter 1. beschriebenen Antikörperstandardlösungen aufgestellt werden kann.
Für die Bestimmung von spezifischem IgE erfolgt eine
relative Quantifizierung über das unter 2. beschriebene Pool-Standard-Serum.

Beispiel 1

Bestimmung von spezifischen Antikörpern der Klasse IgA

a) Herstellung des Konjugats (Antigen)

Äquimolare Mengen von Peptiden der allgemeinen
Formel I und Jodacetylsuccinimidester in Dioxan:
Wasser 1:2 (v:v), enthaltend 100 mmol/l Natriumhydrogencarbonat werden bei 4°C 20 Stunden unter
leichtem Rühren inkubiert. Das Dioxan wird anschließend im Vakuum abgezogen und das gebildete
Reaktionsprodukt aus der wäßrigen Phase durch Ansäuern mit HCl ausgefällt. Nach Waschen in salzsaurem Milieu wird das Produkt lyophilisiert.

100 mg jodacetyliertes Peptid und Trägerprotein, z.B.
250 mg menschliches Serumalbumin werden in 8 molarem
Harnstoff, enthaltend 0,1 mM Natriumbicarbonat,
72 Stunden bei 37°C inkubiert. Nach 3-tägiger Dialyse
gegen destilliertes Wasser (Wechsel der Dialysierflüssigkeit 6 mal) wird das an Albumin gekoppelte
Peptid lyophilisiert. Nach dem beschriebenen Verfahren werden im Mittel 10 Mol Peptid pro Mol Trägerprotein kovalent gebunden.

b) Adsorption des Konjugats an Kunststoffoberflächen,
z.B. Polystyrol

Im vorliegenden Beispiel wurden Polystyrolröhrchen
mit einem Fassungsvermögen von knapp 2 ml, 4 cm hoch,
Durchmesser: 1 cm, verwandt. Zur Bindung eignen sich
auch z.B. Mikrotiterplatten, Kugeln, Küvetten etc.
aus gleichartigem Kunststoffmaterial.

100 µl Konjugatlösung (entsprechend 100 ng Konjugat in 0.1 molarem Bicarbonatpuffer pH 9.6) werden in die Röhrchen einpipettiert und über 24 Stunden bei 37°C getrocknet. Die trockenen Röhrchen werden jeweils einmal mit 200 µl 10 mmol/l Phosphatpuffer pH 7.2 in isotoner Kochsalzlösung, enthaltend 1 % Tween® 80, gewaschen und nach Absaugen der Waschlösung nochmals mit 200 µl destilliertem Wasser gespült und wieder abgesaugt. Die mit dem Konjugat beschichteten Röhrchen werden im Trockenschrank bei 37°C aufbewahrt.

c) <u>Durchführung des Testes</u>

100 µl spezifische Standardlösung (siehe Standardherstellung) oder Serum mit bekanntem Gehalt an spezifischem IgA werden in die wie oben beschrieben beschichteten Röhrchen einpipettiert und 2 Stunden bei 20°C inkubiert. Untersuchungen von biologischem Material, z.B. Patientenseren, werden, um in den Meßbereich der Standardkurve zu gelangen, je nach Titer 1:5 bis 1:500 mit PBS*/Tween® 80-Puffer verdünnt und in gleichem Volumen eingesetzt. Der Inkubationsansatz wird abgesaugt und anschließend zweimal mit 200 µl PBS/Tween® 80-Puffer gewaschen und wieder abgesaugt. 100 µl (Antihuman-IgA)-Immunglobulin (z.B. von der Ziege), markiert mit Peroxidase oder alkalischer Phosphatase, werden in die Röhrchen einpipettiert und 1 Stunde bei konstanter Temperatur zwischen 20°C und 37°C inkubiert. Anschließend wird nochmals zweimal mit je 200 µl PBS/Tween®-Puffer gewaschen. Bei Verwendung des Indikatorsystems konjugierte Peroxidase werden als Substrat o-Phenylendiamin und $H_2O_2$ in Citratpuffer 0,1 mol/l, pH 5.0, zugesetzt und 30 Minuten bei konstanter Temperatur inkubiert.

* phosphate buffered saline

Zum Stoppen der Reaktion werden 50 µl 5 normale H$_2$SO$_4$ zugesetzt. Die Extinktion bei 492 nm ist proportional zur eingesetzten Immunglobulinmenge.

Beispiel 2

Bestimmung von spezifischen Antikörpern der Klasse IgM

a) Herstellung des Konjugats (Antigen)

Die Herstellung des Konjugats erfolgt wie in Beispiel 1 beschrieben.

b) Adsorption des Konjugats an Kunststoffoberflächen, z.B. Polystyrol

Auch die Adsorption des Konjugats an Kunststoffoberflächen verläuft wie in Beispiel 1 beschrieben mit der Abänderung, daß anstelle von 100 ng Konjugat 50 ng Konjugat in 0,1 molarem Bicarbonat-Puffer (pH 9,6) verwendet werden.

c) Durchführung des Tests

Vorbehandlung der Seren: Zum Ausschluß von Interferenzen durch "Rheumafaktoren" werden die Seren vor Verwendung im Testsystem an Latex-gebundenem IgG (z.B. Latex-RF-Reagenz ®) behandelt.

100 µl spezifische Standardlösung (siehe Standardherstellung) oder Serum mit bekanntem Gehalt an spezifischem IgM werden in die wie oben beschrieben beschichteten Röhrchen einpipettiert und 2 Stunden bei 20°C inkubiert. Untersuchungen von biologischem Material, z.B. Patientenseren, werden, um in den Meßbereich der Standardkurve zu gelangen,

je nach Titer mit 0,3 mol/l Glycin-NaCl-Puffer
pH 8,2 verdünnt und in gleichem Volumen eingesetzt.
Der Inkubationsansatz wird abgesaugt und anschließend
zweimal mit 200 μl 0,3 mol/l Glycin-NaCl-
Puffer pH 8,2 gewaschen und wieder abgesaugt.
100 μl (Antihuman-IgM)-Immunglobulin (z.B. von der
Ziege), markiert mit Peroxidase oder alkalischer
Phosphatase, werden in die Röhrchen einpipettiert
und 1 Stunde bei konstanter Temperatur zwischen 20°C
und 37°C inkubiert. Anschließend wird nochmals zweimal
mit je 200 ul 0,3 mol/l Glycin-NaCl-Puffer
pH 8,2 gewaschen. Bei Verwendung des Indikatorsystems
konjugierte Peroxidase werden als Substrat o-Phenylendiamin und $H_2O_2$ in Citratpuffer 0,1 mol/l, pH 5,0,
zugesetzt und 30 Minuten bei konstanter Temperatur
inkubiert.

Zum Stoppen der Reaktion werden 50 μl 5 normale
$H_2SO_4$ zugesetzt. Die Extinktion bei 492 nm ist
proportional zur eingesetzten Immunglobulinmenge.

Beispiel 3

Bestimmung von spezifischen Antikörpern der Klasse IgE

a) Herstellung des Konjugats (Antigen)

Die Herstellung des Konjugats erfolgt wie in
Beispiel 1 beschrieben.

b) Adsorption des Konjugats an Kunststoffoberflächen,
z.B. Polystyrol

Auch die Adsorption des Konjugats an Kunststoffoberflächen verläuft wie in Beispiel 1 beschrieben mit der Abänderung, daß anstelle von 100 ng Konjugat 200 ng Konjugat in 0,1 molarem Bicarbonat-Puffer (pH 9,6) verwendet werden.

c) <u>Durchführung des Tests</u>

Patientenseren werden, um in den Meßbereich des Standard-Poolserums zu gelangen, unverdünnt bis 1:20 verdünnt eingesetzt.
100 µl Serum werden dabei mit 100 µl PBS/Tween® 80-Puffer in die wie oben beschrieben beschichteten Röhrchen einpipettiert und 3 Stunden bei 20°C inkubiert. Der Inkubationsansatz wird abgesaugt und anschließend zweimal mit 200 µl PBS/Tween® 80-Puffer gewaschen und wieder abgesaugt. 100 µl (Antihuman-IgE)-Immunglobulin (z.B. vom Pferd), markiert mit Peroxidase oder alkalischer Phosphatase, werden in die Röhrchen einpipettiert und 2 Stunden bei konstanter Temperatur zwischen 20°C und 37°C inkubiert. Anschließend wird nochmals zweimal mit je 200 µl PBS/Tween® 80-Puffer gewaschen.
Bei Verwendung des Indikatorsystems konjugierte Peroxidase werden als Substrat o-Phenylendiamin und $H_2O_2$ in Citratpuffer 0,1 mol/l, pH 5,0, zugesetzt und 30 Minuten bei konstanter Temperatur inkubiert.

Zum Stoppen der Reaktion werden 50 µl 5 normale $H_2SO_4$ zugesetzt. Die Extinktion bei 492 nm ist proportional zur eingesetzten Immunglobulinmenge.

Gewinnung eines spezifischen humanen Immunglobulin-A-
Standards

1. Kopplung des Albumin-(L-Ala-D-Glu(L-Lys-D-Ala-D-Ala))-
konjugats an Sepharose 4 B

des Konjugats
Die Synthese erfolgt wie für den Test beschrieben.
10 ml Sepharosegel werden fünfmal mit 4- bis
5-fachem Überschuß an destilliertem Wasser gewaschen
und dann in 10 ml Wasser suspendiert. Dazu werden
430 mg Bromcyan, gelöst in 8 ml Wasser, gegeben.
Die Reaktion findet bei Raumtemperatur statt, der
pH-Wert wird auf 11,0 durch Zugabe von 2 mol/l
Natronlauge konstant gehalten. Nach 15-minütiger
Reaktion unter leichtem Rühren wird das Gel auf
einen Büchnertrichter abgesaugt und mit 60 ml
0,1 mol/l Natriumhydrogencarbonatlösung nachgewaschen. Die Sepharose wird in 7,5 ml 0,1 mol/l
Natriumhydrogencarbonat, die 50 mg Albumin-Peptidkonjugat enthalten, eingerührt. Die Kopplung
erfolgt über Nacht bei 4$^o$C unter leichtem Rühren.
Nach der Reaktionszeit wird wieder auf der Filternutsche abgesaugt und das gekoppelte Gel jeweils
zweimal mit 30 bis 40 ml Wasser, dann 0,1 mol/l
Natriumhydrogencarbonat, 0,2 mol/l Natriumacetatpuffer pH 4,5, 0,2 mol/l Natriumphosphatpuffer
pH 7,2 und 0,01 mol/l PBS pH 7,2 gewaschen.
Das Affinitätsgel wird in PBS-Puffer pH 7,2 bei 4$^o$C
aufgehoben.

2. Durchführung der Affinitätschromatographie

Das Gel wird in eine passende Säule gefüllt und
antikörperhaltiges Serum bzw. eine isolierte Immunglobulin-A-fraktion aufgegeben. Die beschickte Säule wird mit PBS-Puffer (ohne Tween) gewaschen bis die
Extinktion bei 280 nm im Eluat unter 0,03 absinkt
(Verbrauch ca. die doppelte Menge Puffer wie Serum).

Die Elution erfolgt mit Essigsäure (0,1 - 1 mol/l).
Der Antikörper wird mit den ersten Fraktionen der
Essigsäure eluiert, die antikörperhaltigen Fraktionen
werden vereinigt, neutralisiert, dialysiert und
konzentriert.

Alternativ kann eine Standardisierung nach folgendem
Verfahren erfolgen:
Die Konzentration an spezifischem IgA in einem Pool-
Serum wird mit Hilfe der Affinitätschromatographie
bestimmt. Dazu wird die isolierte IgA-Fraktion aus
diesem Pool-Serum auf die Säule aufgegeben und
gewaschen, wie oben beschrieben. Die vollständige
Elution des spezifisch gebundenen IgA erfolgt mit
6 mol/l Guanidiniumchlorid. Nach Dialyse wird der
Proteingehalt des Eluats bestimmt.

Bestimmung des Gehalts an spezifischem IgM in einem
Standard-Pool-Serum

1. Kopplung des Albumin-(L-Ala-D-Glu(L-Lys-D-Ala-D-Ala))-
konjugats an Sepharose 4 B

Die Synthese erfolgt wie für den Test beschrieben.
10 ml Sepharosegel werden fünfmal mit 4- bis 5-fachem
Überschuß an destilliertem Wasser gewaschen und dann
in 10 ml Wasser suspendiert. Dazu werden 430 mg
Bromcyan, gelöst in 8 ml Wasser, gegeben. Die Reaktion
findet bei Raumtemperatur statt, der pH-Wert wird
auf 11,0 durch Zugabe von 2 mol/l Natronlauge konstant
gehalten. Nach 15-minütiger Reaktion unter leichtem Rühren wird das Gel auf einen Büchnertrichter abgesaugt und mit 60 ml 0,1 mol/l Natriumhydrogencarbonatlösung nachgewaschen. Die Sepharose wird in 7,5 ml
0,1 mol/l Natriumhydrogencarbonat, die 50 mg Albumin-
Peptid-konjugat enthalten, eingerührt. Die Kopplung
erfolgt über Nacht bei 4°C unter leichtem Rühren.
Nach der Reaktionszeit wird wieder auf der Filternutsche abgesaugt und das gekoppelte Gel jeweils
zweimal mit 30 bis 40 ml Wasser, dann 0,1 mol/l
Natriumhydrogencarbonat, 0,2 mol/l Natriumacetatpuffer pH 4,5, 0,2 mol/l Natriumphosphatpuffer
pH 7,2 und 0,01 mol/l PBS pH 7,2 gewaschen. Das
Affinitätsgel wird in PBS-Puffer pH 7,2 bei 4°C
aufgehoben.

2. <u>Durchführung der Affinitätschromatographie</u>

Das Gel wird in eine passende Säule gefüllt und
isoliertes IgM aufgegeben. Die beschickte Säule
wird mit PBS-Puffer (ohne Tween) gewaschen, bis die
Extinktion bei 280 nm Eluat unter 0,03 absinkt
(Verbrauch ca. die doppelte Menge Puffer wie Serum).

Die vollständige Elution des spezifisch gebundenen
IgM erfolgt mit 6 mol/l Guanidiniumchlorid. Nach
Dialyse wird der Proteingehalt des Eluats bestimmt.

Patentansprüche

1. Antikörper aus der Klasse der Immunglobuline G gegen
das Peptiduntereinheit-Pentapeptid L-Ala-D-Glu(L-Lys-
D-Ala-D-Ala) des Peptidoglycans in biologischem
Untersuchungsmaterial sowie gegen diese Peptidoglycane
enthaltende bakterielle Zellwände mit

a) spezifischer Bindung an Pentapeptide der allgemeinen
Formel

$$R - A_1 - D\text{-}Ala \qquad (I)$$

worin

R  ein Aminosäureanteil mit 1 - 3, vorzugsweise
3 Aminosäuren aus der Gruppe Gly, L-Ala,
D-Glu, L-Lys und

$A_1$  eine der Aminosäuren D-Ala, D-Ser, D-Val oder
D-Leu bedeuten, oder

b) spezifischer Bindung an Pentapeptide der allgemeinen
Formel I enthaltende isolierte bakterielle Zellwände
oder

c) spezifischer Bindung an Pentapeptide der allgemeinen
Formel I enthaltende grampositive Bakterien gemäß
DBP ... (deutsche Patentanmeldung P 32 31 204.0),
gekennzeichnet durch Antikörper aus der Klasse
der Immunoglobuline A, M und E.

2. Reagenz zur Bestimmung von Antikörpern aus der Klasse
der Immunoglobuline A, M und E gegen das Peptiduntereinheit-Pentapeptid L-Ala-D-Glu(L-Lys-D-Ala-D-Ala) des
Peptidoglycans in biologischem Untersuchungsmaterial
sowie gegen diese Peptidoglycane enthaltende bakterielle
Zellwände gemäß Anspruch 1, dadurch gekennzeichnet,
daß eine Verbindung der allgemeinen Formel

$$R - A_1 - D-Ala \qquad (I)$$

worin

R ein Aminosäureanteil mit 1 - 3, vorzugsweise 3 Aminosäuren aus der Gruppe Gly, L-Ala, D-Glu, L-Lys und

$A_1$ eine der Aminosäuren D-Ala, D-Ser, D-Val oder D-Leu
bedeutet,

an einen festen Träger gekoppelt ist und ein spezifischer Antikörper-Standard zur Quantifizierung verwendet wird.

3. Verfahren zur quantitativen Bestimmung von Antikörpern
aus der Klasse der Immunoglobuline A, M und E gegen
das Peptiduntereinheit-Pentapeptid L-Ala-D-Glu(L-Lys-
D-Ala-D-Ala) des Peptidoglycans in biologischem Untersuchungsmaterial sowie gegen diese Peptidoglycane
enthaltende bakterielle Zellwände gemäß Anspruch 1
nach an sich bekannten immunchemischen Verfahren,
dadurch gekennzeichnet, daß man als Antigen eine
Verbindung der allgemeinen Formel

$$R - A_1 - D-Ala \qquad (I)$$

worin

R ein Aminosäureanteil mit 1 - 3, vorzugsweise
3 Aminosäuren aus der Gruppe Gly, L-Ala, D-Glu,
L-Lys und

$A_1$ eine der Aminosäuren D-Ala, D-Ser, D-Val oder
D-Leu bedeutet, verwendet.

4. Verfahren nach Anspruch 3, dadurch gekennzeichnet,
daß die quantitative Bestimmung von Antikörpern in
einem Doppel-Antikörper-Radioimmun-Assay durchgeführt
wird.

5. Verfahren nach Anspruch 3, dadurch gekennzeichnet,
daß die quantitative Bestimmung von Antikörpern in
einem Doppel-Antikörper-Enzymimmun-Assay durchgeführt wird.

6. Verfahren nach Anspruch 3, dadurch gekennzeichnet,
daß die quantitative Bestimmung von Antikörpern
mittels einer nephelometrischen Methode durchgeführt
wird.

7. Verfahren nach Anspruch 3, dadurch gekennzeichnet,
daß die quantitative Bestimmung von Antikörpern in
einem Festphasen-Sandwich-Radioimmun-Assay durchgeführt wird.

8. Verfahren nach Anspruch 3, dadurch gekennzeichnet,
daß die quantitative Bestimmung von Antikörpern
in einem Festphasen-Enzymimmun-Assay durchgeführt
wird.

9. Verfahren nach Anspruch 3, dadurch gekennzeichnet,
daß die quantitative Bestimmung von Antikörpern in
einem Festphasen-Fluoreszenz-Immun-Assay durchgeführt wird.

10. Verfahren nach Anspruch 3, dadurch gekennzeichnet,
daß die quantitative Bestimmung von Antikörpern
mittels eines Latex-Agglutinationstests durchgeführt
wird.

11. Verfahren nach Anspruch 3 zur quantitativen Bestimmung von Antikörpern aus der Klasse der Immunglobuline
A gegen das Peptiduntereinheit-Pentapeptid L-Ala-
D-Glu(L-Lys-D-Ala-D-Ala) des Peptidoglycans in
biologischem Untersuchungsmaterial sowie gegen diese
Peptidoglycane enthaltende bakterielle Zellwände, dadurch gekennzeichnet, daß ein durch Affinitätschromatographie an Sepharose-(Albumin-Peptid der
Formel I) dargestellter spezifischer IgA-Standard
verwendet wird.

12. Verfahren zur quantitativen Bestimmung von Antikörpern
der IgA-Klasse gemäß Anspruch 3, dadurch gekennzeichnet, daß eine isolierte IgA-Fraktion durch
Affinitätschromatographie an Sepharose-(Albumin-
Peptid der Formel I) quantifiziert wird.

13. Verfahren nach Anspruch 3 zur quantitativen Bestimmung von Antikörpern der Klasse IgM gegen das
Peptiduntereinheit-Pentapeptid L-Ala-D-Glu(L-Lys-
D-Ala-D-Ala) des Peptidoglycans in biologischem
Untersuchungsmaterial sowie gegen diese Peptidoglycane enthaltende bakterielle Zellwände, dadurch
gekennzeichnet, daß eine isolierte IgM-Fraktion
durch Affinitätschromatographie an Sepharose-(Albumin-
Peptid der Formel I) quantifiziert wird.